# EUROPEAN PATENT SPECIFICATION

(11) **EP 4 259 036 B1**
(45) Date of publication and mention of the grant of the patent: **02.12.2026**
(21) Application number: 21907402.8
(22) Date of filing: 19.10.2021
(51) Int. Cl.: A61C 17/06, A61C 17/10, A61C 17/08, A61C 17/14

(54) **DENTAL MOUTHPIECES**
ZAHNMUNDSTÜCKE
EMBOUTS BUCCAUX DENTAIRES

(30) Priority: 14.12.2020 US 202063125182 P; 22.07.2021 US 202117383258
(43) Date of publication of application: 18.10.2023
(73) Proprietor: Zyris, Inc., Santa Barbara, CA 93117 (US)
(72) Inventor: KERR, Amy, Santa Barbara, California 93117 (US); ONTIVEROS, Adam, Santa Barbara, California 93117 (US); HALLECK, Brian, Santa Barbara, California 93117 (US); SWARTZ, Jacob, Santa Barbara, California 93117 (US); HIRSCH, James, Santa Barbara, California 93117 (US); VALENTINE, Shaun, Santa Barbara, California 93117 (US)
(74) Representative: Script Intellectual Property LLP
(86) International application number: PCT/US2021/055627
(87) International publication number: WO 2022/132302

(56) References cited:
- US-A- 5 037 298
- US-A1- 2005 051 178
- US-A1- 2007 218 422
- US-A1- 2010 119 989
- US-A1- 2010 119 989
- US-A1- 2010 304 324
- US-B2- 10 292 800
- US-B2- 9 532 858

## Description

### BACKGROUND OF THE INVENTION

### 1. Field of the Invention

Embodiments of the invention relate generally to dental mouthpieces. More particularly, embodiments of the invention relate to a dental mouthpiece having features of retraction, suction, tongue guard, airway protection, suction options, and the like.

### 2. Description of Prior Art and Related Information

The following background information may present examples of specific aspects of the prior art (e.g., without limitation, approaches, facts, or common wisdom) that, while expected to be helpful to further educate the reader as to additional aspects of the prior art, is not to be construed as limiting the present invention, or any embodiments thereof, to anything stated or implied therein or inferred thereupon.

In performing dental procedures, there is often a need to perform multiple functions at the same time. Such functions can include keeping the patient's mouth open, retracting tissue such as a patient's lips, providing appropriate suction, and the like. Conventional lip and cheek retractors may be uncomfortable for users and do not provide any additional features or protections for the patient or doctor.

US2010304324A1 describes a bite block comprising a sheet having a rectangular center body portion with opposing wings attached to lateral sides of the center body portion. Adjacent the lateral sides are fold lines by which the wings may be folded towards each other. When inserted in the mouth, the bite block holds the mouth open while the opposing wings protect the cheeks.

US2007218422A1 describes an adjustable cheek retractor with saliva extracting tongue guard. The tongue guard comprises holders to secure a perforated vacuum tubing through which saliva is extracted from a patient's mouth.

US2010119989A1 describes a lip and cheek retracting device comprising a front wall, a rear wall and a bottom portion defining a continuous concave channel utilized to retract a patient's lips. The retractor has a bite block. An aspiration block connected to the bite block has an aspiration inlet and is connected to vacuum tubing for aspiration of fluid from the patient's tongue.

In view of the foregoing, there is a need for improved dental mouthpieces that can provide multiple features that can benefit both the patient and the doctor.

### SUMMARY OF THE INVENTION

The present invention provide a dental mouthpiece comprising lip retractor portions at opposing ends of the mouthpiece; a central region having a bite block member on each side thereof; cheek retractor portions connecting the lip retractor portions to the central region; at least one suction connector at the central region, characterized in that the dental mouthpiece includes: at least one suction opening disposed along a width of the central region, the at least one suction opening communicating with the at least one suction connector, wherein suction provided at the at least one suction connector provides a suction at the at least one suction opening; a suction channel formed across the width of the central region when the dental mouthpiece is in use, the suction channel providing a flow path between the at least one suction connector and the at least one suction opening, the at least one suction opening formed along the suction channel; and suction openings disposed at distal ends of the suction channel.

These and other features, aspects and advantages of the present invention will become better understood with reference to the following drawings, description and claims.

### BRIEF DESCRIPTION OF THE DRAWINGS

Some embodiments of the present invention are illustrated as an example and are not limited by the figures of the accompanying drawings, in which like references may indicate similar elements.
FIG. 1 illustrates front view of an anterior mouthpiece design according to an exemplary embodiment of the present invention;
FIG. 2 illustrates a back view of the mouthpiece design of FIG. 1;
FIG. 3 illustrates a side view of the mouthpiece design of FIG. 1;
FIG. 4 illustrates a side view, bent into an insertion / in use configuration, of the mouthpiece of FIG. 1;
FIG. 5 illustrates a front view of a semi-rigid frame anterior mouthpiece design according to an exemplary embodiment of the present invention;
FIG. 6 illustrates a rear perspective view of the mouthpiece of FIG. 5;
FIG. 7 illustrates a front view of a soft flexible material molded over a semi-rigid frame anterior mouthpiece design according to an exemplary embodiment of the present invention;
FIG. 8 illustrates a side view of the mouthpiece of FIG. 7;
FIG. 9 illustrates a side view, bent into an insertion / in use configuration, of the mouthpiece of FIG. 7;
FIG. 10 illustrates a front view of a mouthpiece according to an exemplary embodiment of the present invention;
FIG. 11 illustrates a back view of the mouthpiece of FIG. 10;
FIG. 12 illustrates a side view of the mouthpiece of FIG. 10;
FIG. 13 illustrates an end view of the mouthpiece of FIG. 10;
FIG. 14 illustrates a front view of the mouthpiece of FIG. 10 in a natural state; and
FIG. 15 illustrates a front view of the mouthpiece of FIG. 10 in a flexed state.

Unless otherwise indicated illustrations in the figures are not necessarily drawn to scale.

The invention and its various embodiments can now be better understood by turning to the following detailed description wherein illustrated embodiments are described. It is to be expressly understood that the illustrated embodiments are set forth as examples and not by way of limitations on the invention as ultimately defined in the claims.

### DETAILED DESCRIPTION OF THE PREFERRED EMBODIMENTS AND BEST MODE OF INVENTION

The terminology used herein is for the purpose of describing particular embodiments only and is not intended to be limiting of the invention. As used herein, the term "and/or" includes any and all combinations of one or more of the associated listed items. As used herein, the singular forms "a," "an," and "the" are intended to include the plural forms as well as the singular forms, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

Unless otherwise defined, all terms (including technical and scientific terms) used herein have the same meaning as commonly understood by one having ordinary skill in the art to which this invention belongs. It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

In describing the invention, it will be understood that a number of techniques and steps are disclosed. Each of these has individual benefit and each can also be used in conjunction with one or more, or in some cases all, of the other disclosed techniques. Accordingly, for the sake of clarity, this description will refrain from repeating every possible combination of the individual steps in an unnecessary fashion. Nevertheless, the specification and claims should be read with the understanding that such combinations are entirely within the scope of the invention and the claims.

In the following description, for purposes of explanation, numerous specific details are set forth in order to provide a thorough understanding of the present invention. It will be evident, however, to one skilled in the art that the present invention may be practiced without these specific details.

The present disclosure is to be considered as an exemplification of the invention and is not intended to limit the invention to the specific embodiments illustrated by the figures or description below.

As is well known to those skilled in the art, many careful considerations and compromises typically must be made when designing for the optimal configuration of a commercial implementation of any system, and in particular, the embodiments of the present invention. A commercial implementation in accordance with the spirit and teachings of the present invention may be configured according to the needs of the particular application, whereby any aspect(s), feature(s), function(s), result(s), component(s), approach(es), or step(s) of the teachings related to any described embodiment of the present invention may be suitably omitted, included, adapted, mixed and matched, or improved and/or optimized by those skilled in the art, using their average skills and known techniques, to achieve the desired implementation that addresses the needs of the particular application.

Broadly, embodiments of the present invention provide a dental mouthpiece that can provide multiple features that can benefit both the patient and the doctor. The mouthpiece can take a generally flat configuration when not in use and may be resiliently folded into a C-shape for insertion into the patient's mouth. In some embodiments, the frame may be rigid or semi-rigid. The mouthpiece may provide retraction, suction ability, tongue control, airway and throat protection and a bite block in a single disposable or reusable device.

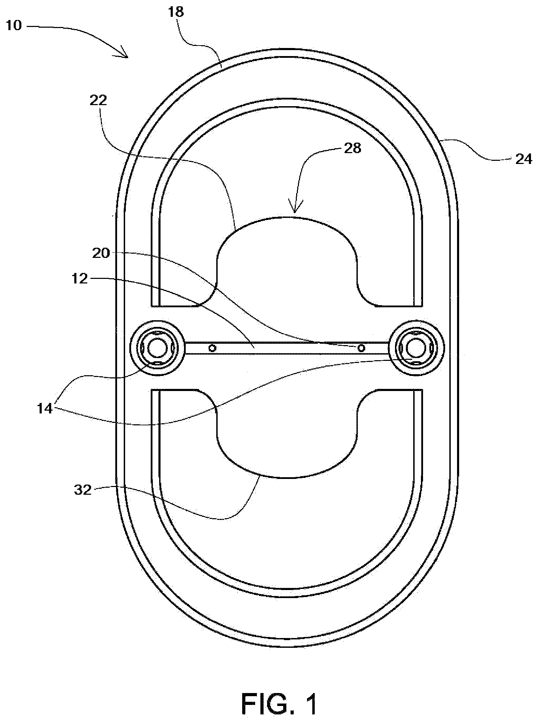

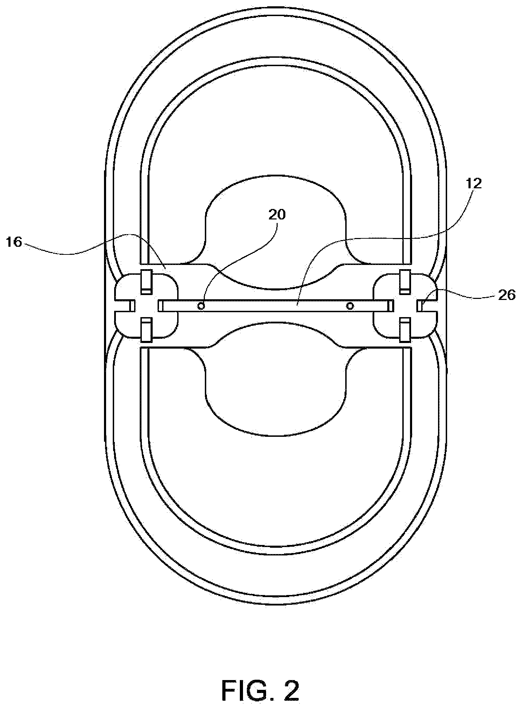

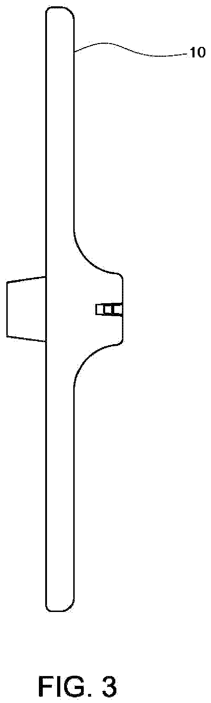

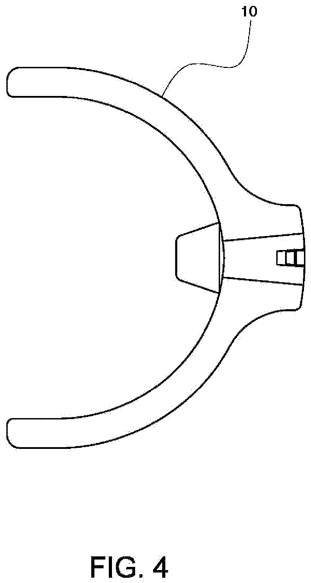

Referring now to FIGS. 1 through 4, according to a first embodiment of the present invention, a mouthpiece 10 can include suction channels 12, where, once the device is placed in the mouth, by bending the device into a C-shape, the mouthpiece 10 creates the suction channel 12 which provides suction when at least one of the suction ports 14 are connected to external suction. The suction can be provided, via the suction channels 12 at, for example, in the area of a bite block 16 and lip retractors 18.

Suction ports 20 communicate with the suction channel 12 so that saliva and excess irrigation fluid can be evacuated from the mouth and to provide inhibition from contaminating treatment areas of the oral cavity in the active treatment areas. Suction connector port 14 can include elastic materials to allow for connection with variety of sizes and designs of external suction sources. Suction can be provided in the airway protection 22, the bite block 16, the flexural support members 24, and the lip retractors 18, for example.

Suction openings 26 in the oral cavity have features to inhibit tissue from fully blocking suction. The suction openings 26 provide suction to a buccal region of the patient when the mouthpiece is in place. When in use, the airway protection portion 22 and tongue shield 32 can keep a patient's tongue downward and out of the way. The user can rest their jaw on the bite block 16 while the lip retractors 18 can keep the lips out of the way from any procedures being performed by the dentist or hygienist, for example.

In the mouthpiece, the flexural resistance can assist in the insertion for various mouth morphologies where flexural support members 24 of the mouthpiece 10 open after folding for insertion and consistently self-seats the device in the maxillary and mandibular buccal vestibules of each patient.

The lip retractors 18 can extend outward from the central core of the device. These elements can include the flexural members which, when positioned, provide elevation and retraction for the labial surfaces both maxillary and mandibular. The counteractive forces of the maxillary and mandibular support members keep the lips retracted and away from treatment areas.

The molded flexural support members 24 and the lip retractors 18 provide upper lip, lower lip, and cheek retraction. This retraction provides unobstructed access and visibility for facial/labial areas of the mouth during dental procedures. The lips are prevented from touching gingival tissue and tooth structure. Lips and soft tissue are retracted and held away from dental tools for improved safety.

Flexural support members 24 can be joined to the central core 28 of the device by a molded connection 30. Gussets or fillets can be added to the flexural support member, thus positioning the bending center further away from the central core to create more retraction or lift to the lip retractor 4 as required.

A tongue shield 32 can inhibit patients from extending their tongue forward past the bite block 16, discouraging a patient from interfering during dental procedures and maintaining safety for the patient.

The airway protection portion 22 can extends up to the upper palatal vault and a throat protective shield 32 (also referred to as tongue shield 32) can extend to the floor of the mouth, thereby inhibiting debris from entering the patients throat and airway thus inhibiting accidental aspiration or ingestion of debris or equipment.

One or more external suction connection ports 14 allows for clinical access from multiple working positions without removal or reinsertion of the device into the oral cavity. While the device shown in FIGS. 1 through 4 include two such suction ports 14, the number and/or position of such ports may vary.

The bilateral bite block 16 can provide a place for patients to rest their jaw as well as providing an interincisal opening allowing for lingual, buccal, and occlusal access on the mandibular and maxillary arch.

The device of FIGS. 1 through 4 can provide an integrated retractor, bite block, tongue shield, airway shield and suction module in a disposable unit or in a non-disposable unit, depending on user needs and the specific application.

The entire device can be made of a soft material, similar to gingival tissue, to allow for comfort against the gums. For example, the device can be made from soft flexible materials, such as plastics, rubber, foams or the like. The device can include molded bumpers to allow the lip retractors to enhance patient comfort by inhibiting impingement directly against gingival tissue. In some embodiments, the bumpers may be formed from a sponge-like material to permit absorption of excess irrigation fluids from the oral cavity.

As described above, the device can be molded so that the device is substantially flat or semi-flat in a storage, non-use condition to allow for simple mold designs. The device can be resiliently confirmed into a C-shape bent configuration during oral insertion and during use thereof.

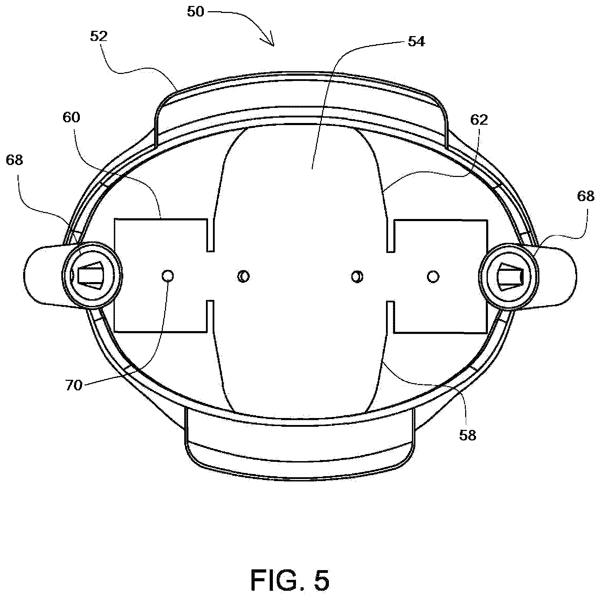

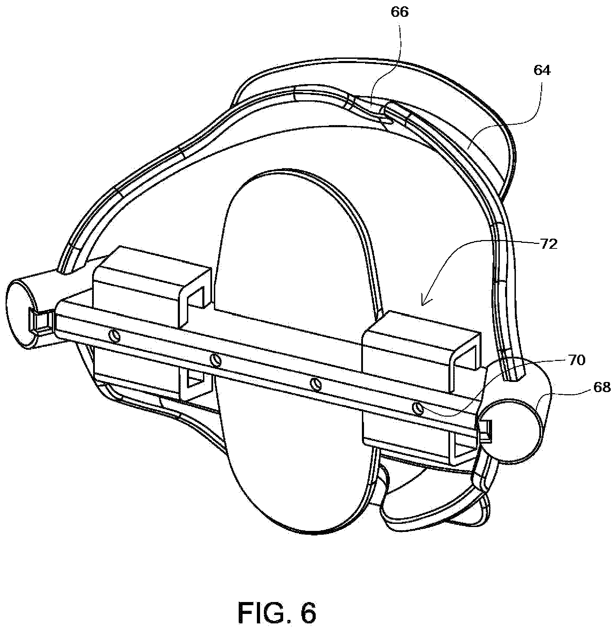

Referring now to FIGS. 5 and 6, a semi-rigid frame anterior mouthpiece 50 is shown. The features may be similar to many of those described above and as further discussed below.

Labial retraction can be provided by lip retractors 52 of the device that extend outward from the central core 54 of the device. These elements can include flexural members which, when positioned, provide elevation and retraction for the labial surfaces, both maxillary and mandibular. The counteractive forces of the maxillary and mandibular support members keep the lips retracted and away from treatment areas.

The molded flexural support members 56 provide upper lip, lower lip, and cheek retraction. This retraction provides unobstructed access and visibility for facial/labial areas of the mouth during dental procedures. The lips are prevented from touching gingival tissue and tooth structure. Lips and soft tissue are retracted and held away from dental tools for improved safety.

Flexural support members can be joined to the central core of the device by a molded connection. Gussets or fillets are added to the flexural support member positioning the bending center further away from the central core thus creating more retraction or lift to the lip retractor areas as required.

The tongue shield 58 can inhibit patients from extending their tongue forward past the bite block 60, discouraging a patient from interfering during dental procedures maintaining safety for patient.

The airway and throat protective shield 62 can extend up to the upper palatal vault and down to the floor of the mouth, thereby inhibiting debris from entering the patients throat and airway thus inhibiting accidental aspiration or ingestion of debris or equipment.

Molded retraction frame provides a self-seating lip saddle 64 that can guide the placement of the lips and inhibit them from becoming dislodged. Upper and lower cut out 66 can allow for frenum comfort.

The mouthpiece provides one or more suction ports 68 which, when connected to external suction provide a channel for saliva and excess irrigation fluids to be evacuated from the mouth and inhibits contaminating treatment areas of the oral cavity in the active treatment areas. One or more external suction connection ports 68 allow for clinical access from multiple working positions without removal or reinsertion of the device into the oral cavity. The suction openings 70 in the oral cavity can include features to inhibit tissue from fully blocking suction. Suction connector ports 68 can be formed from an elastic material to allow for connection with variety of sizes and designs of external suction sources.

Suction can be provided in the airway protection, bite block, flexural support members, lip retractors, suction channels, and external suction ports.

The bilateral bite blocks 60 provide a place for patients to rest their jaw as well as providing an interincisal opening allowing for lingual, buccal, and occlusal access on the mandibular and maxillary arch. The molded bite block module 72 is designed to be attached to the semi-rigid frame with a detailed mechanism feature which inhibits improper attachment and orientation. The molded bite block module 72 / tongue shield unit 58 can connect to the frame orientation by various means, such as a feature that guides the connection and permits use of the mouthpiece with either the guard in the frame or removed from the frame and out of the patient's mouth.

The device provides a retractor, bite block, tongue shield, airway shield and suction module in a disposable unit or a non-disposable unit, depending on the particular application.

The device can include molded bumpers to allow the lip retractors to enhance patient comfort by inhibiting impingement directly against gingival tissue. In some embodiments, the bumpers may be formed from a sponge-like material to permit absorption of excess irrigation fluids from the oral cavity.

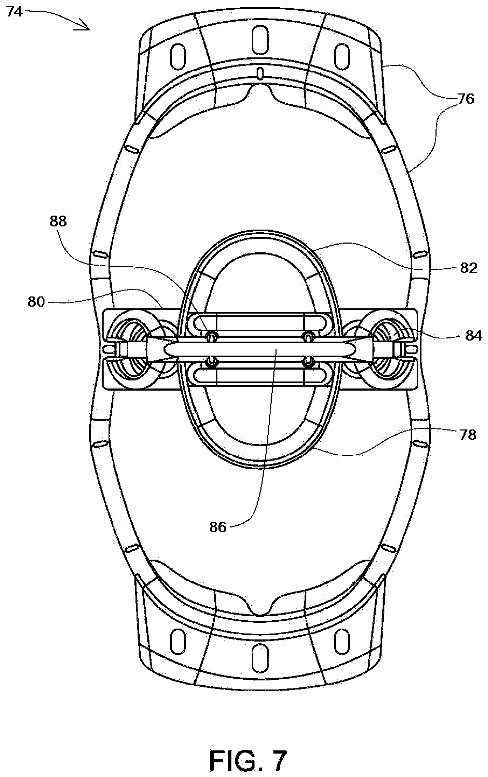

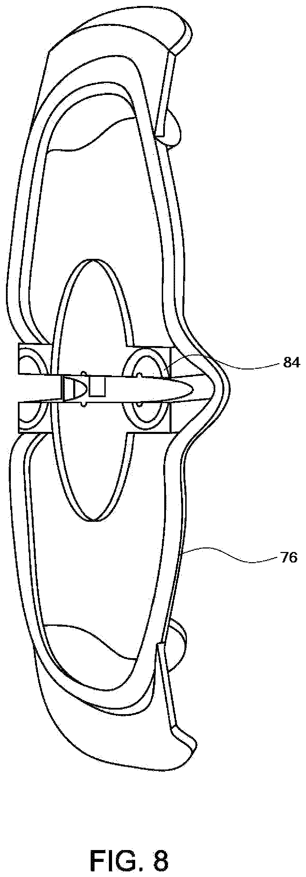

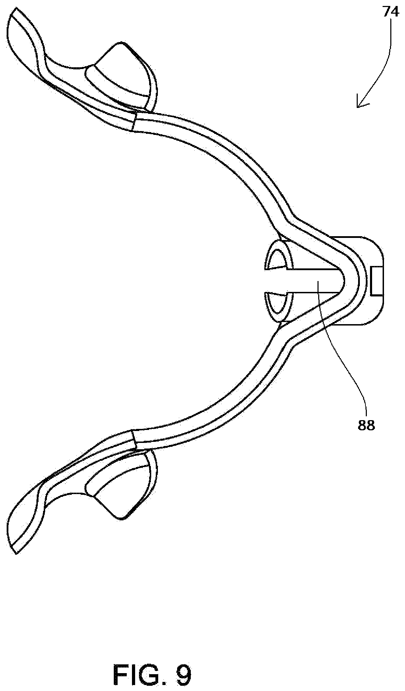

Referring now to FIGS. 7 through 9, a flexible material molded over a semi-rigid frame anterior mouthpiece system 74 is shown. The molded flexural support members 76 provide upper lip, lower lip, and cheek retraction. This retraction provides unobstructed access and visibility for facial/labial areas of the mouth during dental procedures. The lips can be prevented from touching gingival tissue and tooth structure. Lips and soft tissue can be retracted and held safely away from dental tools.

Labial retraction is provided by elements of the device that extend outward from the central core of the device, these elements include flexible members 76 which, when positioned in the use position, provide elevation and retraction for the labial surfaces both maxillary and mandibular. The counteractive forces of the maxillary and mandibular support members keep the lips retracted and away from treatment areas.

Support members will be joined to the central core of the device by a molded connection. Gussets or fillets are added to the support member positioning the bending center further away from the central core thus creating more retraction or lift to the lip retractor areas as required. Flexural resistance assists in the retraction for multiple mouth morphologies where flexural support members of mouthpiece open after folding for insertion and self-seats device in the maxillary and mandibular vestibules.

One or more materials are used to create a rigid back bone for retraction and a soft outer structure for comfort. Molded structure made of one or more dissimilar materials provide for retraction and patient comfort with minimal flexural members. For example, the mouthpiece can be made from a semi-rigid core over-molded with a soft, flexible material.

The device of FIGS. 7 through 9 may include features similar to that of the above structures, including those specifically described below. The tongue shield 78 can prevent patients from extending their tongue forward past the bite block 80, preventing a patient from interfering during dental procedures maintaining safety for patient.

The airway protection 82 can extend up to the upper palatal vault and down to the floor of the mouth which prevents debris from entering the patients throat and airways thus preventing accidental aspiration or ingestion of debris or equipment.

The bilateral bite blocks 80 provide a place to rest their jaw as well as providing an interincisal opening allowing for lingual, buccal, and occlusal access on the mandibular and maxillary arch.

The mouthpiece provides one or more suction ports 84 which, when connected to external suction, provides a channel 86 for saliva and excess irrigation fluids to be evacuated from the mouth. Once placed in the mouth, the mouthpiece will create suction channel 86 which provide suction when connected to external suction. Suction will be provided in the airway protection, bite block, flexural support members, suction channels, and suction ports.

The distributed suction openings 8 in the oral cavity have features to prevent tissue from fully blocking suction.

External suction connector port 84 can be formed from an elastic material to allow for connection with external suction device. One or more external suction connection ports 84 allow for clinical access from multiple working positions.

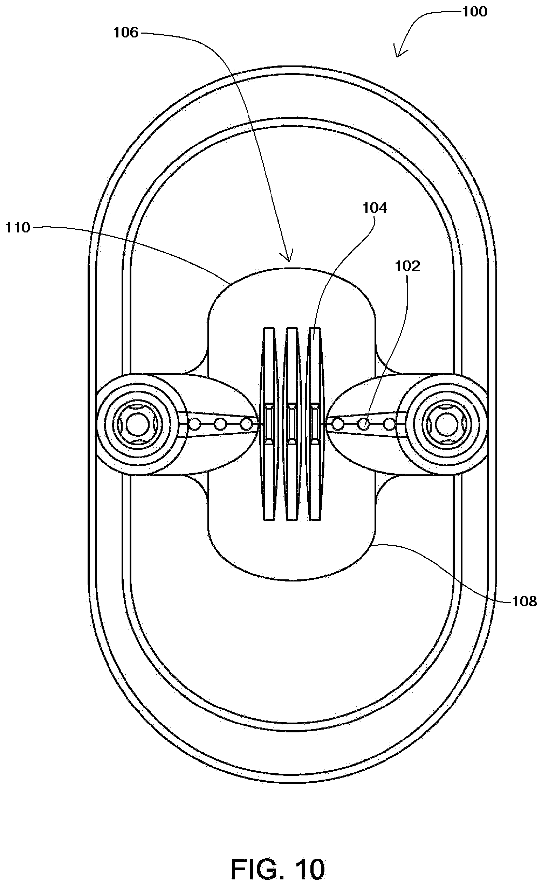

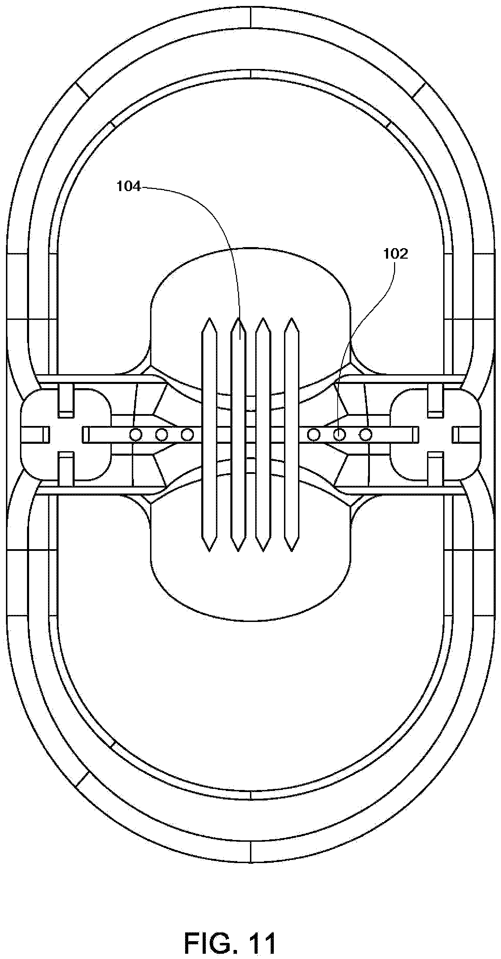

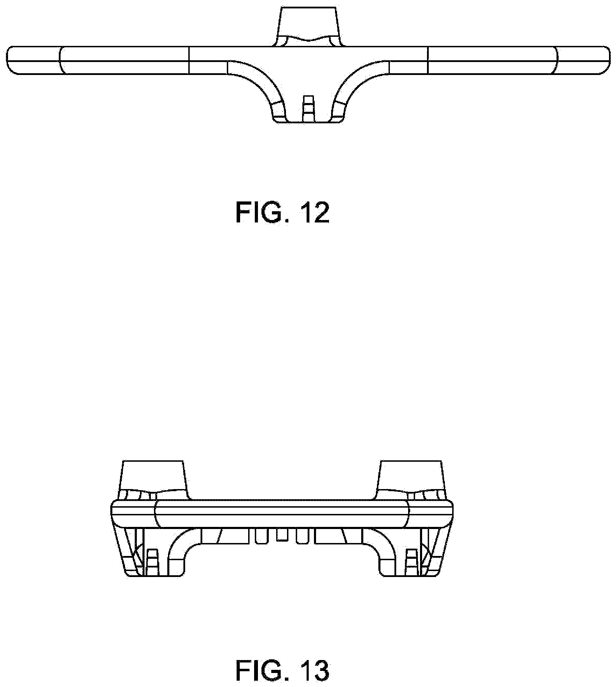

The embodiment of FIGS. 10-15 illustrate suction ports 102 along a width of the device 100. Such suction ports 102 can provide a negative pressure in the mouth of the patient, avoiding excess flow of droplets from the patient's mouth into the surrounding atmosphere. Such negative pressure may be especially useful for preventing the spread of virus from the patient to the dental professional.

The device 100 may provide features similar to the mouthpieces described above, where, when bent into a C-shape, it can be inserted into the patient's mouth to provide a bite block, lip and cheek retraction, and suction. The Embodiment of FIGS. 10-15 differ from that described above in that a central region 106, which includes a tongue guard 108 and an airway protection portion 110, has a plurality of thinned slots 104 formed therein.

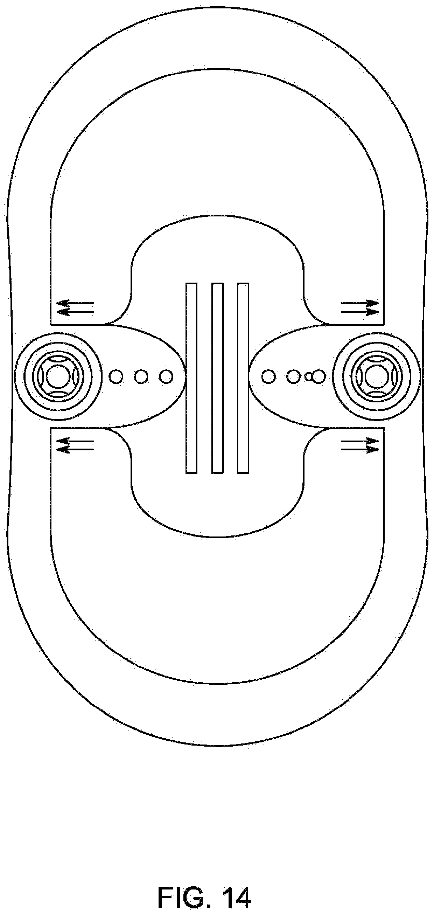

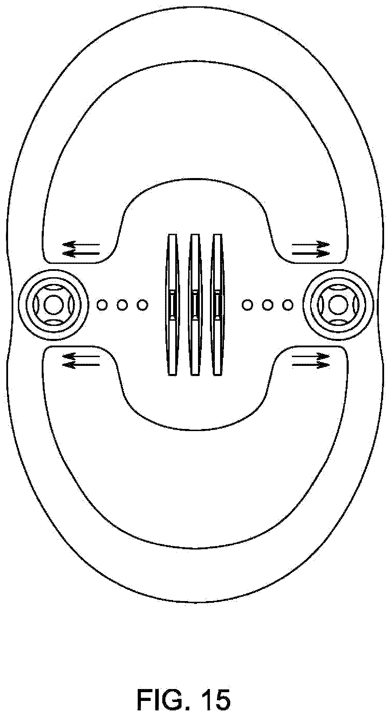

FIGS. 14 and 15 illustrate how the size of the device may be flexed to adjust to various sized oral cavities. The central region 106 may include regions of reduced thickness 104 to permit stretching outward or pressing inward of the sides of the device. In some embodiments, the reduced thickness regions may be slits through the material, but in other embodiments, as shown, the reduced thickness regions may be solid and reduced a given length and width to permit width-wise adjustability, as shown. Such adjustability may be from about 0.5 to about 3 cm, for example.

The devices described above provide retractor, bite block, tongue shield, airway shield and suction module in a disposable unit or a non-disposable unit, depending on the particular application.

The device can include molded bumpers to allow the lip retractors to enhance patient comfort by inhibiting impingement directly against gingival tissue. **In** some embodiments, the bumpers may be formed from a sponge-like material to permit absorption of excess irrigation fluids from the oral cavity.

The device can be molded so that the device is substantially flat or semi-flat in a storage, non-use condition to allow for simple mold designs. The device can be resiliently confirmed into a C-shape bent configuration during oral insertion and during use thereof. Such bending can close the channel, creating a tunnel for suction. Structure can flex to allow for external suction insertion and has flexural retraction to secure external suction in the mouthpiece.

The entire device can be made of a soft material similar to gingival tissue to allow for comfort against gums.

## Claims

1. A dental mouthpiece comprising:
lip retractor portions (18) at opposing ends of the mouthpiece;
a central region (106) having a bite block member on each side thereof;
cheek retractor portions (24) connecting the lip retractor portions to the central region; and
at least one suction connector (14) at the central region, **characterized in that** the dental mouthpiece includes:
at least one suction opening (20) disposed along a width of the central region, the at least one suction opening communicating with the at least one suction connector, wherein suction provided at the at least one suction connector provides a suction at the at least one suction opening;
a suction channel (12) formed across the width of the central region by bending the dental mouthpiece into a C-shape when the dental mouthpiece is in use, the suction channel providing a flow path between the at least one suction connector and the at least one suction opening (20), the at least one suction opening (20) formed along the suction channel; and
further suction openings (26) disposed at distal ends of the suction channel.

2. The dental mouthpiece of claim 1, wherein the lip retractor portions, cheek retractor portions and central region are coplanar in a non-use state of the dental mouthpiece.

3. The dental mouthpiece of claim 2, wherein the cheek retractor is flexible to permit the dental mouthpiece to fold into a C-shaped, use state thereof.

4. The dental mouthpiece of claim 1, wherein the central region includes a tongue guard extending downward toward a tongue of the patient during use of the dental mouthpiece.

5. The dental mouthpiece of claim 1, wherein the central region includes an airway protection portion extending upward during use of the dental mouthpiece.

6. The dental mouthpiece of claim 1, further comprising expansion and contraction elements disposed in the central region, the expansion and contraction elements permitting a width of the central region to be adjustable.

7. The dental mouthpiece of claim 6, wherein the expansion and contraction elements are reduced thickness channels formed lengthwise in the central region.

8. The dental mouthpiece of claim 7, wherein the reduced thickness channels include at least one slot cut therethrough.

9. The dental mouthpiece of claim 1, further comprising lip saddles disposed on each of the lip retractors.

10. The dental mouthpiece of claim 9, further comprising a cutout in the lip saddle for frenum comfort during use of the dental mouthpiece.

11. The dental mouthpiece of claim 6, wherein:
the lip retractor portions (18) are formed as arcs at opposing ends of the mouthpiece;
the cheek retractor portions (24) connect each end of the arc of each of the lip retractor portions to the central region; and
the mouthpiece comprises a plurality of suction openings (20) disposed in both a front side and a rear side along a width of the central region, the plurality of suction openings communicating with the at least one suction connector, wherein suction provided at the at least one suction connector provides a suction at the plurality of suction openings.

12. The dental mouthpiece of claim 11, wherein the lip retractor portions, cheek retractor portions and central region are coplanar in a non-use state of the dental mouthpiece, and wherein the cheek retractor is flexible to permit the dental mouthpiece to fold into a C-shaped, use state thereof.

13. The dental mouthpiece of claim 11, wherein the central region includes a tongue guard, extending downward toward a tongue of the patient during use of the dental mouthpiece, and an airway protection portion extending upward during use of the dental mouthpiece.

## Patentansprüche

1. Zahnmedizinisches Mundstück umfassend:
Lippenhalterabschnitte (18) an entgegengesetzten Enden des Mundstücks;
einen Mittelbereich (106), der auf jeder Seite desselben ein Beißblockelement aufweist;
Wangenhalterabschnitte (24), welche die Lippenhalterabschnitte mit dem Mittelbereich verbinden; und
mindestens einen Sauganschluss (14) am Mittelbereich, **dadurch gekennzeichnet, dass** das zahnmedizinische Mundstück Folgendes einschließt:
mindestens eine Saugöffnung (20), die entlang einer Breite des Mittelbereichs angeordnet ist, wobei die mindestens eine Saugöffnung mit dem mindestens einen Sauganschluss in Verbindung steht, wobei die an dem mindestens einen Sauganschluss bereitgestellte Saugwirkung eine Saugwirkung an der mindestens einen Saugöffnung bereitstellt;
einen Saugkanal (12), der über die Breite des Mittelbereichs durch Biegen des zahnmedizinischen Mundstücks in eine C-Form geformt ist, wenn das zahnmedizinische Mundstück in Gebrauch ist, wobei der Saugkanal einen Strömungsweg zwischen dem mindestens einen Sauganschluss und der mindestens einen Saugöffnung (20) bereitstellt, wobei die mindestens eine Saugöffnung (20) entlang des Saugkanals geformt ist; und
weitere Saugöffnungen (26), die an distalen Enden des Saugkanals angeordnet sind.

2. Zahnmedizinisches Mundstück nach Anspruch 1, wobei die Lippenhalterabschnitte, die Wangenhalterabschnitte und der Mittelbereich in einem Nichtgebrauchszustand des zahnmedizinischen Mundstücks komplanar sind.

3. Zahnmedizinisches Mundstück nach Anspruch 2, wobei der Wangenhalter flexibel ist, um zu ermöglichen, dass sich das zahnmedizinische Mundstück in einen C-förmigen Gebrauchszustand desselben faltet.

4. Zahnmedizinisches Mundstück nach Anspruch 1, wobei der Mittelbereich einen Zungenschutz einschließt, der sich während des Gebrauchs des zahnmedizinischen Mundstücks nach unten in Richtung einer Zunge des Patienten erstreckt.

5. Zahnmedizinisches Mundstück nach Anspruch 1, wobei der Mittelbereich einen Atemwegsschutzabschnitt einschließt, der sich während des Gebrauchs des zahnmedizinischen Mundstücks nach oben erstreckt.

6. Zahnmedizinisches Mundstück nach Anspruch 1, ferner umfassend Expansions- und Kontraktionselemente, die im Mittelbereich angeordnet sind, wobei die Expansions- und Kontraktionselemente ermöglichen, dass eine Breite des Mittelbereichs angepasst wird.

7. Zahnmedizinisches Mundstück nach Anspruch 6, wobei die Expansions- und Kontraktionselemente Kanäle mit reduzierter Dicke sind, die in Längsrichtung im Mittelbereich geformt sind.

8. Zahnmedizinisches Mundstück nach Anspruch 7, wobei die Kanäle mit reduzierter Dicke mindestens einen durchgeschnittenen Schlitz einschließen.

9. Zahnmedizinisches Mundstück nach Anspruch 1, ferner umfassend Lippensättel, die auf jedem von den Lippenhaltern angeordnet sind.

10. Zahnmedizinisches Mundstück nach Anspruch 9, ferner umfassend einen Ausschnitt im Lippensattel für Komfort des Lippenbändchens während des Gebrauchs des zahnmedizinischen Mundstücks.

11. Zahnmedizinisches Mundstück nach Anspruch 6, wobei:
die Lippenhalterabschnitte (18) als Bögen an entgegengesetzten Enden des Mundstücks geformt sind;
die Wangenhalterabschnitte (24) jedes Ende des Bogens jedes von den Lippenhalterabschnitten mit dem Mittelbereich verbinden und
das Mundstück eine Vielzahl von Saugöffnungen (20) umfasst, die in sowohl einer Vorderseite als auch einer Rückseite entlang einer Breite des Mittelbereichs angeordnet sind, wobei die Vielzahl von Saugöffnungen mit dem mindestens einen Sauganschluss in Verbindung steht, wobei die an dem mindestens einen Sauganschluss bereitgestellte Saugwirkung eine Saugwirkung an der Vielzahl von Saugöffnungen bereitstellt.

12. Zahnmedizinisches Mundstück nach Anspruch 11, wobei die Lippenhalterabschnitte, die Wangenhalterabschnitte und der Mittelbereich in einem Nichtgebrauchszustand des zahnmedizinischen Mundstücks komplanar sind und wobei der Wangenhalter flexibel ist, um zu ermöglichen, dass sich das Mundstück in einen C-förmigen Gebrauchszustand desselben faltet.

13. Zahnmedizinisches Mundstück nach Anspruch 11, wobei der Mittelbereich einen Zungenschutz, der sich während des Gebrauchs des zahnmedizinischen Mundstücks nach unten in Richtung einer Zunge des Patienten erstreckt, und einen Atemwegsschutzabschnitt, der sich während des Gebrauchs des zahnmedizinischen Mundstücks nach oben erstreckt, einschließt.

## Revendications

1. Embout buccal dentaire comprenant :
des parties d'écarteur des lèvres (18) aux extrémités opposées de l'embout buccal ;
une région centrale (106) présentant un élément de bloc de morsure de chaque côté ;
des parties d'écarteur des joues (24) reliant les parties d'écarteur des lèvres à la région centrale ; et
au moins un connecteur d'aspiration (14) au niveau de la région centrale, **caractérisé en ce que** l'embout buccal dentaire inclut :
au moins une ouverture d'aspiration (20) disposée le long d'une largeur de la région centrale, l'au moins une ouverture d'aspiration communiquant avec l'au moins un connecteur d'aspiration, dans lequel l'aspiration fournie au niveau de l'au moins un connecteur d'aspiration fournit une aspiration au niveau de l'au moins une ouverture d'aspiration ;
un canal d'aspiration (12) formé sur la largeur de la région centrale en pliant l'embout buccal dentaire en forme de C lorsque celui-ci est utilisé, le canal d'aspiration assurant une voie d'écoulement entre l'au moins un connecteur d'aspiration et l'au moins une ouverture d'aspiration (20), l'au moins une ouverture d'aspiration (20) étant formée le long du canal d'aspiration ; et
d'autres ouvertures d'aspiration (26) disposées aux extrémités distales du canal d'aspiration.

2. Embout buccal dentaire selon la revendication 1, dans lequel les parties d'écarteur des lèvres, les parties d'écarteur des joues et la région centrale sont coplanaires lorsque l'embout buccal dentaire n'est pas utilisé.

3. Embout buccal dentaire selon la revendication 2, dans lequel l'écarteur de joues est flexible pour permettre à l'embout buccal dentaire de se plier en forme de C, pendant son état d'utilisation.

4. Embout buccal dentaire selon la revendication 1, dans lequel la région centrale inclut un protège-langue s'étendant vers le bas en direction de la langue du patient pendant l'utilisation de l'embout buccal dentaire.

5. Embout buccal dentaire selon la revendication 1, dans lequel la région centrale inclut une partie de protection des voies respiratoires s'étendant vers le haut pendant l'utilisation de l'embout buccal dentaire.

6. Embout buccal dentaire selon la revendication 1, comprenant en outre des éléments d'expansion et de contraction disposés dans la région centrale, les éléments d'expansion et de contraction permettant de régler la largeur de la région centrale.

7. Embout buccal dentaire selon la revendication 6, dans lequel les éléments d'expansion et de contraction sont des canaux d'épaisseur réduite formés longitudinalement dans la région centrale.

8. Embout buccal dentaire selon la revendication 7, dans lequel les canaux d'épaisseur réduite incluent au moins une fente découpée à travers eux.

9. Embout buccal dentaire selon la revendication 1, comprenant en outre des selles labiales disposées sur chacun des écarteurs de lèvres.

10. Embout buccal dentaire selon la revendication 9, comprenant en outre une découpe dans la selle labiale pour le confort du frein pendant l'utilisation de l'embout buccal dentaire.

11. Embout buccal dentaire selon la revendication 6, dans lequel :
les parties d'écarteur des lèvres (18) sont formées en arcs aux extrémités opposées de l'embout buccal ;
les parties d'écarteur des joues (24) relient chaque extrémité de l'arc de chacune des parties d'écarteur des lèvres à la région centrale ; et
l'embout buccal comprend une pluralité d'ouvertures d'aspiration (20) disposées à la fois sur une face avant et une face arrière le long d'une largeur de la région centrale, la pluralité d'ouvertures d'aspiration communiquant avec l'au moins un connecteur d'aspiration, dans lequel l'aspiration fournie au niveau de l'au moins un connecteur d'aspiration fournit une aspiration au niveau de la pluralité d'ouvertures d'aspiration.

12. Embout buccal dentaire selon la revendication 11, dans lequel les parties d'écarteur des lèvres, les parties d'écarteur des joues et la région centrale sont coplanaires lorsque l'embout buccal dentaire n'est pas utilisé, et dans lequel l'écarteur des joues est flexible pour permettre à l'embout buccal dentaire de se plier en forme de C, pendant son état d'utilisation.

13. Embout buccal dentaire selon la revendication 11, dans lequel la région centrale inclut un protège-langue s'étendant vers le bas en direction de la langue du patient pendant l'utilisation de l'embout buccal dentaire, et une partie de protection des voies respiratoires s'étendant vers le haut pendant l'utilisation de l'embout buccal dentaire.
